# EUROPEAN PATENT APPLICATION

(11) **EP 1 618 857 A1**
(43) Date of publication of application: **25.01.2006**
(21) Application number: 04730021.5
(22) Date of filing: 28.04.2004
(51) Int. Cl.: A61F 2/16

(54) **SINGLE PIECE INTRAOCULAR LENS AND METHOD FOR PRODUCING SAME**

(30) Priority: 28.04.2003 JP 2003124256
(71) Applicant: HOYA HEALTHCARE CORPORATION, Tokyo 161-0032 (JP)
(72) Inventor: KUROSAKA, Daijiro, Tokyo 158-0085 (JP); SHIBUYA, Akihiko, C/O HOYA HEALTHCARE CORPORATION, Tokyo 161-0032 (JP); MITOMO, Kikuo, C/O HOYA HEALTHCARE CORPORATION, Tokyo 161-0032 (JP); TATSUISHI, Tomomitsu, C/O HOYA HEALTHCARE CORP., Tokyo 161-0032 (JP)
(74) Representative: Grünecker, Kinkeldey, Stockmair & Schwanhäusser Anwaltssozietät
(86) International application number: PCT/JP2004/006149
(87) International publication number: WO 2004/096099

(57) **Abstract**

Single-piece type intraocular lens includes a stepped part provided in a boundary between an optic portion 1 and support portions in the posterior 1b of the optic portion 1, in such a way that the surface of a part shifting to the region of the support portion from the region of the optic portion shifts suddenly toward the anterior of the optic portion.

## Description

### Technical Field

The present invention relates to a single-piece type intraocular lens which is inserted into a lens capsule for correcting visual function after removing the crystalline lens opacified by a cataract, and which is obtained by processing the material integrally molded by an optic portion forming material and a support portion forming material, and a manufacturing method for the same.

### Background Art

Generally, an intraocular lens is constituted of an optic portion serving as a lens, and support portions serving as two arm-shaped supporting members attached to the optic portion for containing the optic portion within a lens capsule and supporting it therein.

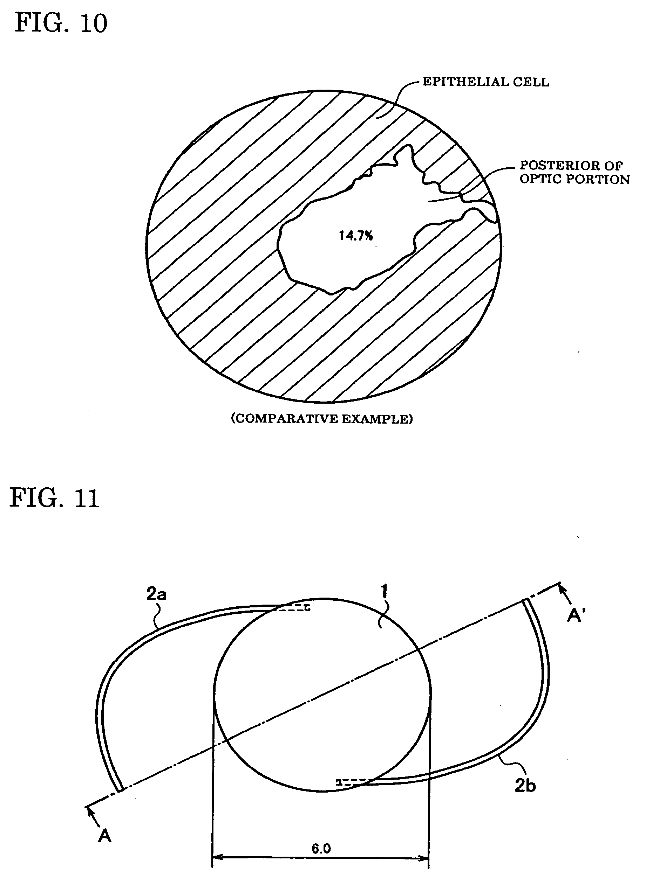

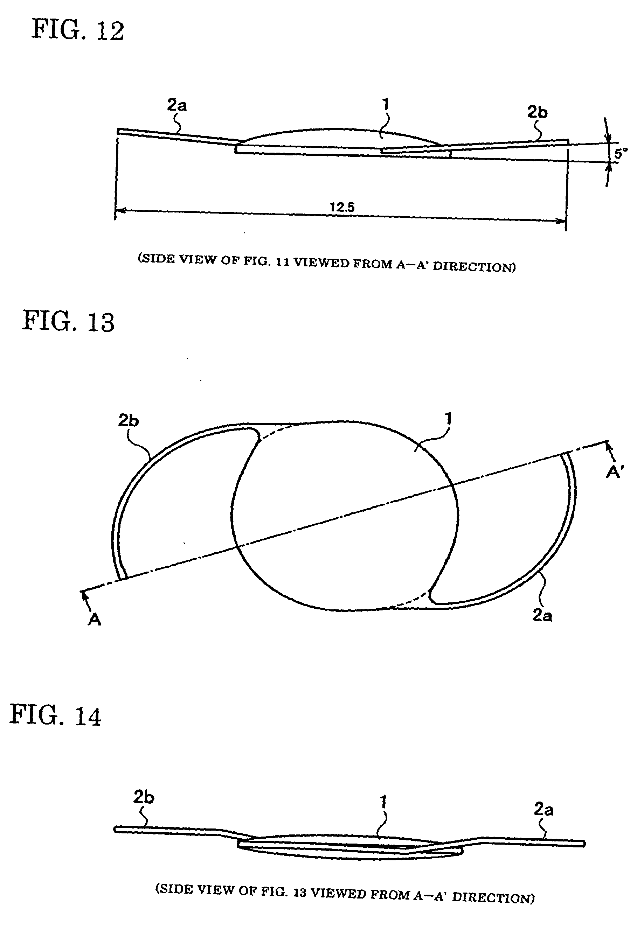

As the intraocular lens described above, a multi-piece type intraocular lens and a single-piece type intraocular lens are known. Fig.11 is a plan view of the conventional multi-piece type intraocular lens, and Fig.12 is a side view of Fig.11. Also, Fig.13 is a plan view of the conventional single-piece type intraocular lens, and Fig.14 is a side view of Fig.13.

As shown in Fig.11 and Fig.12, in the multi-piece type intraocular lens, an optic portion 1 and support portions 2a and 2b are separately made, and thereafter thermally bound together, or joined by laser beam joining method or by means of an adhesive, etc. Meanwhile, as shown in Fig .13 and Fig.14, in the single-piece type intraocular lens, a raw material is manufactured by integrally molding the material forming the optic portion 1 and the material forming the support portions 2a and 2b, and the raw material thus obtained is subjected to pattern formation process by cutting or the like (for example, see Patent Document 1, Patent Document 2, and Patent Document 3).

In this case, conventionally, as a material of the optic portion, polymethyl methacrylate (referred to as PMMA hereafter) having high transparency and exhibiting excellent processing performance has been frequently used. However, in recent years, copolymers of transparent and soft methacrylate and acrylate (referred to as soft acrylic material hereafter) have been frequently used.

The reason is as follows. Specifically, PMMA has a high rigidity and therefore, at least incision having the size larger than the diameter of the optic portion (about 5.5 to 6.5 mm) is required for the insertion of the intraocular lens. Meanwhile, the insertion of the lens made of a soft acrylic material is made possible from smaller incision by folding the soft optic portion, and therefore an advantage is that the soft acrylic material is excellent in minimizing the surgical invation at the time of implantation and the surgery-induced astigmatism.

Further, the soft acrylic material is high in adhesiveness, and therefore it is closely brought into contact with the lens capsule inserted after operation, thus improving positional stability and preventive effect against aftercataract (Lens epithelial cell is allowed to migrate to the posterior of the optic portion to form the fibrosis or swollen and modified cell, with the result that the lens capsule of the posterior of the optic portion is opcified). Therefore, operators have more cases of adopting the intraocular lens made of the soft acrylic material.

Note that as a multi-piece type intraocular lens made of the soft acrylic material, Alcon Acrysof (Trademark) and AMO Sensar (Trademark) etc., are known. In addition, as a single-piece type intraocular lens made of the soft acrylic material, Acryfold (Trademark) developed by the applicants of the present application (HOYA HEALTH CARE CORPORATION) is known.
Patent Document 1 : Japanese Patent Laid open No.08-257046
Patent Document 2 : Japanese Patent Laid open No.11-70130
Patent Document 3 : Japanese Patent Laid open No.11-155944

### Disclosure of the Invention

Meanwhile, if an aftercataract is caused after the operation of inserting an intraocular lens into a lens capsule, a follow-up operation is required. Therefore, the intraocular lens capable of allowing as few generation of the aftercataract as possible is desired. As described above, the aftercataract is caused by allowing the lens epithelial cell to migrate to the back surface side (posterior) of the optic portion to form the fibrosis or swollen and modified cell, with the result that the lens capsule of the back surface side (posterior) of the optic portion is opacified.

Therefore, one of the conceivable effective methods for preventing the generation of the aftercataract is to effectively prevent the lens epithelial cell from migrating to the back surface side (posterior) of the optic portion of the intraocular lens. In this regard, as described above, by making the optic portion with a soft acrylic material having high adhesiveness, and by closely bringing it into contact with the lens capsule, a specific effect can be obtained.

In addition, the single-piece type intraocular lens is manufactured by automatically cutting bulk-polymerized PMMA having a high molecular weight, thereby allowing the material itself to have excellent shape memorizing properties, and in addition, excellent in stability of the intraocular lens after operation and also excellent in productivity. For such reasons, in recent years, the single-piece type intraocular lens has been especially frequently used.

Under such circumstances, operation cases using the single-piece type intraocular lens increase at a faster rate, and accordingly the possibility of the generation of the aftercataract increases probabilistically. Therefore, more complete elimination of the possibility of the aftercataract has been rapidly required.

In order to overcome the above-described background, an object of the present invention is to provide a single-piece type intraocular lens made of the material having excellent shape memorizing properties, superior in stability of the intraocular lens after operation and also superior in productivity, and further capable of remarkably decreasing the possibility of the generation of the aftercataract.

In order to solve the above-described problems, a first aspect of the present invention provides a single-piece type intraocular lens obtained by processing the material which is manufactured by integrally molding an optic portion forming material and a support portion forming material, the single-piece type intraocular lens comprising a stepped part which is provided in a boundary part between the optic portion and the support portions at the posterior of the optic portion in such a way that the surface of a part shifting to the region of the support portions from the region of the optic portion is arranged so as to suddenly shift toward the anterior direction of the optic portion.

A second aspect of the present invention provides the single-piece type intraocular lens according to the first aspect of the present invention, wherein the stepped part has an edge part in a part of the optic portion side in a boundary shifting to the support portions from the optic portion, having a stepped face, e.g. a wall face connecting to the support portions from the edge part in nearly parallel to the optical axis of the optic portion.

A third aspect of the present invention provides the single-piece type intraocular lens according to the second aspect of the present invention, wherein the surface of the posterior of the optic portion near the edge part is formed in a surface substantially orthogonal to the optical axis.

A fourth aspect of the present invention provides the single-piece type intraocular lens according to the second aspect of the present invention, wherein the surface of the posterior of the optic portion near the edge part is formed so as to rise toward the edge part side in the direction of the posterior.

A fifth aspect of the present invention provides the single-piece type intraocular lens according to the second aspect of the present invention, wherein a part of the stepped face closer to the support portions is formed into an acute angle so as to be inclined in the optical axis direction closely to the center of the optical axis.

A sixth aspect of the present invention provides the single-piece type intraocular lens according to the second aspect of the present invention, wherein a part of the stepped face closer to the support portions is formed into an obtuse angle so as to be slightly inclined in the optical axis direction in a direction opposite to the center of the optical axis.

A seventh aspect of the present invention provides the single-piece type intraocular lens according to the second aspect of the present invention, wherein R (curved surface) is formed in a part of the stepped face closer to the support portions.

An eighth aspect of the present invention provides the single-piece type intraocular lens according to the second aspect of the present invention, wherein the stepped face is formed into a concavo-convex face.

A ninth aspect of the present invention provides the single-piece type intraocular lens according to any one of the aspects 1 to 8 of the present invention, wherein the stepped parts have a step difference with heights of 0.05 mm or more.

A tenth aspect of the present invention provides the single-piece type intraocular lens according to any one of the aspects 1 to 9 of the present invention, wherein the edge part of the stepped part located in a region of the optic portion is formed of a soft material.

An eleventh aspect of the present invention provides the single-piece type intraocular lens according to any one of the aspects 1 to 10 of the present invention, wherein the optic portion is formed of a soft material and the support portions are formed of hard materials.

A twelfth aspect of the present invention provides the single-piece type intraocular lens according to the eleventh aspect of the present invention, wherein the soft material is a soft acrylic material, and the hard material is PMMA.

A thirteenth aspect of the present invention provides the single-piece type intraocular lens according to any one of the aspects 1 to 12 of the present invention, wherein the optical surface of the posterior of the optic portion is formed into a concave surface shape.

A fourteenth aspect of the present invention provides a manufacturing method of the single-piece type intraocular lens according to any one of the aspects 1 to 13 of the present invention, comprising:
preparing a raw material which is formed by integrally molding the optic portion forming material and the support portion forming material;
cutting the raw material to form a curved surface shape of the optical surface of both sides of the anterior and the posterior of the optic portion, and form a front surface shape of the support portions located on both sides of the anterior and the posterior of the optic portion;
next, forming a face becoming a stepped face by grooving a part where the stepped part is estimated to be formed; and
next, forming by cutting a contour shape excepting the anterior and posterior of the optic portion, and the contour shape excepting the surface shape located on both sides of the anterior and posterior of the optic portion of the support portion.

According to the above-described aspects of the present invention, by having the stepped parts arranged in such a way that the surface of the part shifting to the region of the support portions from the region of the optic portion shifts suddenly toward the anterior of the optic portion, the edge part formed by the stepped parts is brought into close contact with the lens capsule, thereby completely preventing the lens epithelial cell from migrating toward the posterior of the optic portion by the stepped face serving as a wall.

Further, by forming the front surface of the posterior of the optic portion near the edge part in a surface substantially orthogonal to the optical axis, the edge part is easily brought into close contact with the inner surface of the lens capsule (Third aspect). Also, by forming the front surface of the posterior of the optic portion near the edge part so as to rise toward the edge part in a posterior direction, the edge part is easily and surely brought into close contact with the inner surface of the lens capsule, thereby completely preventing the lens epithelial cell from migrating toward the posterior of the optic portion (Fourth aspect).

Further, by forming the part of the stepped face closer to the support portions into an acute angle so as to be inclined in the optical axis direction closely to the center of the optical axis (Fifth aspect), by forming the part of the stepped face closer to the support portions so as to be slightly inclined in the optical axis direction in a direction opposite to the center of the optical axis (Sixth aspect), by forming R (curved surface) in a part of the stepped face closer to the support portions (Seventh aspect), and by forming the stepped face into a concavo-convex face (Eighth aspect), nearly the same effects with those described above can be obtained.

Moreover, in this case, by setting the heights of the stepped parts to 0.05 mm or more (Ninth aspect), or by forming the edge part located in the region of the optic portion of the stepped parts with a soft material (Tenth aspect), by forming the optic portion with a soft material and forming the support portions with hard materials (Eleventh aspect), or by forming the optical surface of the posterior of the optic portion into a concave surface (Twelfth aspect), more effects can be obtained.

In addition, the above-described single-piece type intraocular lens according to the aspects 1 to 13 of the present invention can be comparatively easily manufactured by a manufacturing method of the single-piece type intraocular lens according to the fourteenth aspect of the present invention.

### Brief Description of the Drawings

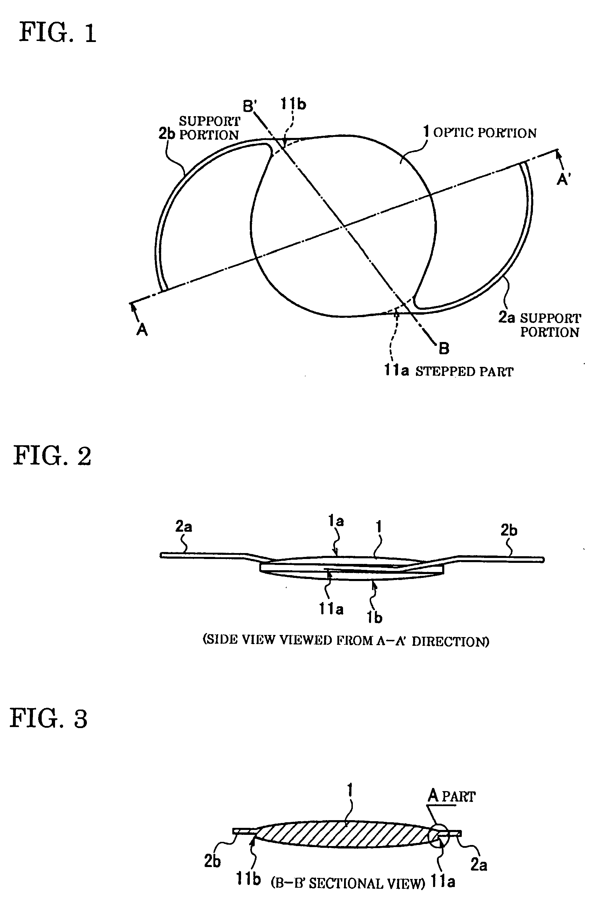

Fig.1 is a plan view of a single-piece type intraocular lens according to an embodiment of the present invention.
Fig.2 is a side view of Fig.1 viewed from AA' direction.
Fig.3 is a sectional view of Fig.1 taken along the line B-B'.

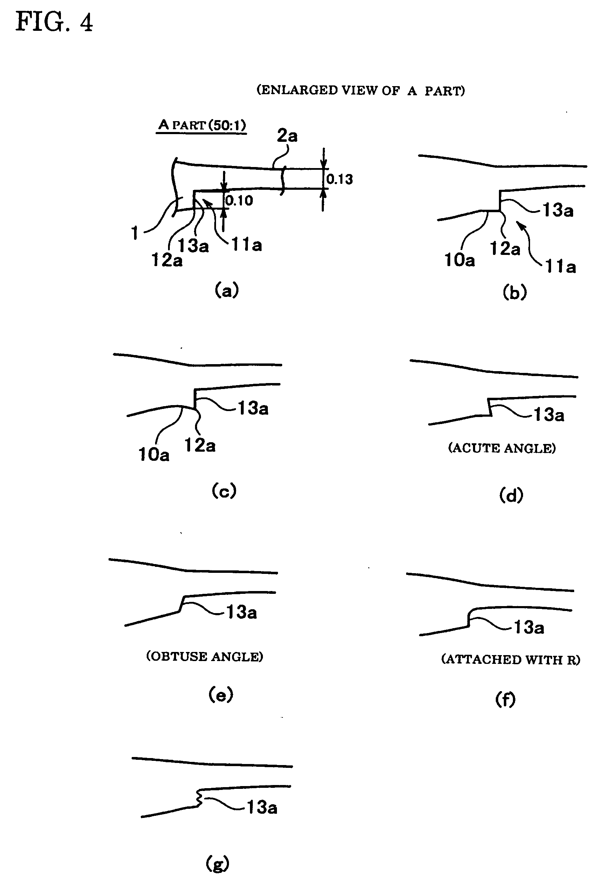

Fig.4 is a view showing a modified example of A part.

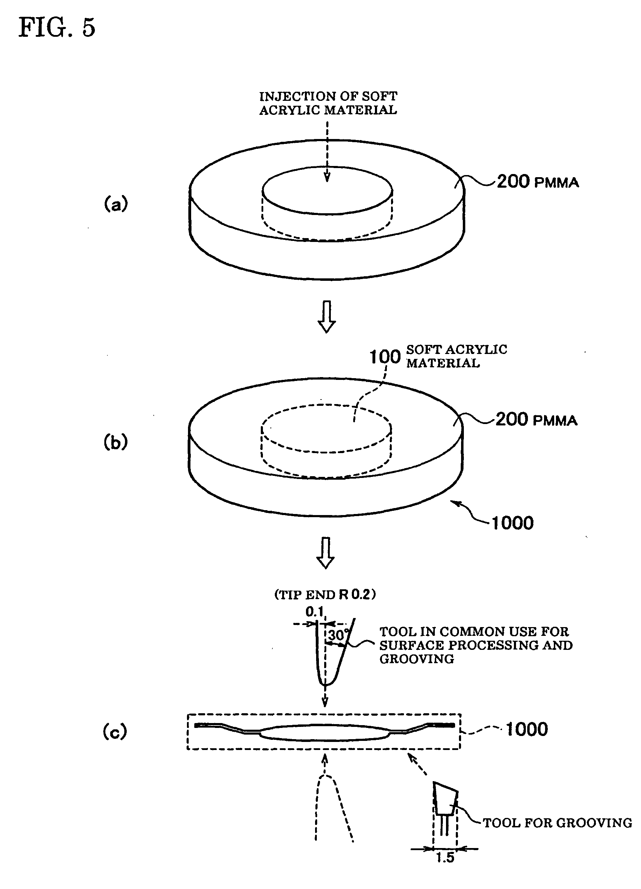

Fig.5 is an explanatory view of a manufacturing method of the single-piece type intraocular lens according to the embodiment of the present invention.

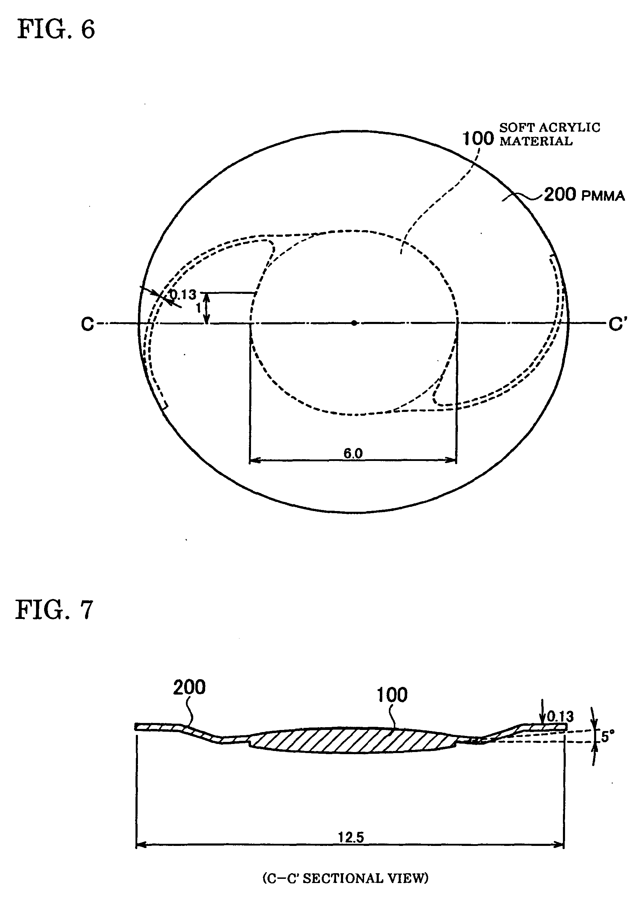

Fig.6 is a plan view of a second intermediate member.
Fig.7 is a sectional view of Fig. 7 taken along the line C-C'.

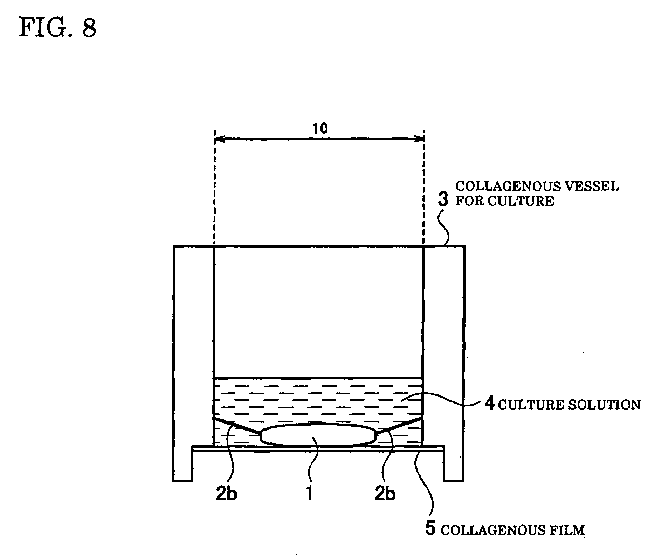

Fig. 8 is an explanatory view of an epithelial cell culture test.

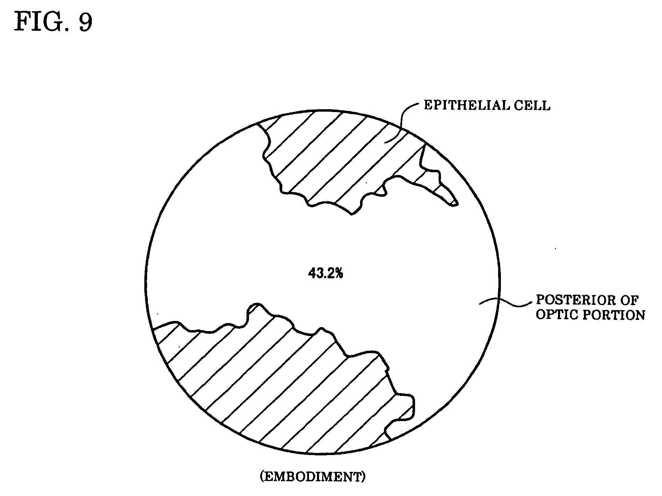

Fig.9 is a view showing a result of the cell culture test of the single-piece type intraocular lens according to the embodiment of the present invention.
Fig.10 is a view showing the result of the cell culture test of the conventional single-piece type intraocular lens.
Fig.11 is a plan view of the conventional multi-piece type intraocular lens.
Fig.12 is a side view of Fig.11.
Fig.13 is a plan view of the conventional single-piece type intraocular lens.
Fig.14 is a side view of Fig.13.
   1 Optic portion
   2 Support portion
   11a Stepped part

### Best Mode for carrying out the Invention

Fig.1 is a plan view of a single-piece type intraocular lens according to an embodiment of the present invention, Fig. 2 is a side view of Fig.1 viewed from AA' direction, Fig.3 is a sectional view of Fig.1 taken along the line B-B', Fig.4(a) is an expanded view of A part of Fig.3, and Figs.4(b) to Fig.4(g) are views showing modified examples of the A part. The single-piece type intraocular lens according to the embodiments of the present invention will be explained with reference to the above-described drawings hereafter.

The single-piece type intraocular lens according to the embodiments of the present invention includes an optic portion 1 formed of a concave lens for correcting the visual function, and support portions 2a and 2b serving as two arm-shaped support members extended toward outside from two positions of the peripheral edge of the optic portion 1 for supporting the optic portion 1, with the optic portion 1 contained in a lens capsule. The optic portion 1 is made of a soft-acrylic material, and the support portions 2a and 2b are made of PMMA, and therefore they are made of different materials but integrally formed.

Moreover, the optic portion 1 has an anterior 1a and a posterior 1b. When the single-piece type intraocular lens is contained in the lens capsule, the anterior 1a is located on the side of a cornea and serves as an optical surface, and the posterior 1b is arranged so as to be in contact with the inner surface of the lens capsule and serves as an optical surface.

On the posterior of the optic portion 1, and in a boundary between the optic portion 1 and the support portions 2a and 2b, stepped parts 11a and 11b are provided respectively. The stepped parts 11a and 11b are arranged in such a way that the surface of the part that shifts to the region of the support portions 2a and 2b from the region of the optic portion 1 suddenly shifts toward the anterior direction of the optic portion 1.

As shown in the sectional enlarged view in Fig.4A, the stepped part 11a has an edge part 12a in a part of the optic portion 1 side in the boundary shifting to the support portions 2a and 2b from the optic portion 1, and a stepped face 13a which is nearly parallel to the optical axis of the optic portion 1 and serves as a wall face connecting to the support portions 2a and 2b from the edge part 12a. In the embodiment, the height of the stepped part 11a is set to be 0.1mm. Note that if the height of the stepped part is set to be 0.05mm or more, a constant effect can be obtained. Also, the edge part 12a is made of a soft acrylic material which is a material forming the optic portion 1.

Further, the stepped part 11a may be the modified examples shown in Figs.4(b) to Fig.4(g). In the example shown in Fig.4(b), a front surface 10a of the posterior 1b of the optic portion 1 near the edge part 12a is formed on a surface substantially orthogonal to an optical axis. With this structure, the edge part 12a is easily in close contact with the inner surface of the lens capsule. In the example shown in Fig.4(c), the front surface 10a of the posterior 1b of the optic portion 1 near the edge part 12a is formed so as to rise toward the edge part 12a in a posterior direction. With this structure, the edge part 12a is easily and surely brought into close contact with the inner surface of the lens capsule, and thereby it is possible to completely prevent the lens epithelial cell from migrating to the posterior of the optic portion.

Further, the example shown in Fig.4(d) shows that a part of the stepped face 13a closer to the support portions 2a and 2b is formed into an acute angle so as to be inclined in the direction of the optical axis closely to the center of the optical axis. The example shown in Fig.4(e) shows that the part of the stepped face 13a closer to the support portions 2a and 2b is formed into an obtuse angle so as to be slightly inclined in the direction of the optical axis in a direction opposite to the center of the optical axis. The example shown in Fig.4(f) shows that R (curved surface) is formed in a part of the stepped face 13a closer to the support portions 2a and 2b. The example shown in Fig.4(g) shows that the stepped face 13a is formed into a concavo-convex face.

Fig.5 is an explanatory view of a manufacturing method of the single-piece type intraocular lens according to the embodiments of the present invention. The manufacturing method of the single-piece type intraocular lens according to the embodiments of the present invention will be explained with reference to Fig.5 hereafter.

First, as shown in Fig.5(a), by using a known forming method, a support portion forming member 200 made of PMMA and formed into substantially a doughnut shape is obtained. Next, a raw material solution becoming a soft acrylic material after cured is injected into a hole part of the center of the support portion forming member 200, and is allowed to be cured therein. Thus, as shown in Fig.5(b), a disc- shaped raw material 1000 is obtained by integrally molding a material 100 forming the optic portion 1 and a material 200 forming the support portions 2a and 2b.

Next, as shown in Fig.5(c), by using a precision lathe having a cutting tool for surface process, the anterior and posterior of the disc-shaped raw material 1000 is subjected to a surface process. That is, the curved surface shape of the optical surfaces of both sides of the anterior 1a and the posterior 1b of the optic portion 1, and the front surface shape of the support portions 2a and 2b located on both sides of the anterior and the posterior of the optic portion 1 are formed. By this process, a first intermediate member is formed. The first intermediate member has a curved surface shape on the anterior and posterior but still is formed in a disc-shape in plan view and has no support portions 2a and 2b formed therein yet.

Next, the first intermediate member is subjected to grooving by the precision lathe equipped with a grooving tool along a circle with the center on the optical axis of the optic portion 1 and passing the boundary part between the region of the optic portion and the region of the support portions 2a and 2b. Thus, a second intermediate member is obtained. In the second intermediate member, an edge-like part and a stepped face becoming an edge part 12a and a stepped face 13a, and a step-like part becoming a stepped part 11a are formed. (See Fig.5(c)). Note that if using a tool in common use for surface processing and grooving, the grooving can be done as a sequence of processes after the surface processing. Dimensions for the tool in common use for surface processing and grooving and the tool for grooving are shown in the figure. Note that the grooving may not be performed in lathe processing, but may be performed by a milling apparatus, when forming the contour shape by the milling apparatus as will be described later.

Fig.6 is a plan view of the second intermediate member, and Fig.7 is a sectional view of Fig.6 taken along the line C-C'. Note that C-C' section in Fig. 6 is a sectional view when cutting the second intermediate member by an arbitrary plan surface including the optical axis of the optic portion 1. Note that the dotted line in Fig. 6 shows a shape of the single-piece type intraocular lens in plan view at the time of completion of the intraocular lens shown in Fig.1. The dimension of each part is shown in the figure. The second intermediate member is processed by using the milling apparatus to obtain the contour shape in plan view shown by the dotted line of Fig. 6, and further is chamfered if necessary to obtain the single-piece type intraocular lens shown in Figs.1 to 3.

Next, an aftercataract prevention capability of the single-piece type intraocular lens thus obtained was checked by the cell culture test as described below. First, a collagenous film vessel 3 (Product of KOKEN CO, LTD. CM-24) having a collagenous film 5 formed at the bottom is prepared. Next, keeping the aseptic condition, and in a state of compressing the support portion to the whole diameter 10mm, the lens is contained in the vessel, with the posterior of the optic portion 1 being in contact with the collagenous film 5 of the bottom. Next, a culture solution 4 was injected into the collagenous film vessel 3, and a bovine lens epithelial cell was sowed on the anterior of the optic portion 1, which was then cultured for eight days.

After cultured for eight days, the single-piece type intraocular lens was taken out from the collagenous film vessel 1, the posterior of the optic portion 1 was checked, and the area not covered with the epithelial cell was measured. If the area is largely covered with cell, it means that few suppressing effects can be expected against the cell invasion to the posterior of the optic portion 1, and also means that the prevention capability against the generation of the aftercataract is inferior. Meanwhile, as the area not covered with cell increases, the prevention capability against the generation of the aftercataract is superior.

The same cell culture test was conducted for the conventional single-piece type intraocular lens shown in Fig.13 and Fig.14, to obtain a comparative example. Fig.9 is a view showing a result of the cell culture test of the single-piece type intraocular lens according to an embodiment of the present invention, and Fig.10 is a view showing a result of the cell culture test of the conventional single-piece type intraocular lens, to obtain the comparative example. From Fig.9 and Fig.10, the single-piece type intraocular lens according to the embodiments has 43.2 % of area not covered with the epithelial cell, while the conventional single-piece type intraocular lens has 14.7 % of area not covered with the epithelial cell. Therefore, it is found that the single-piece type intraocular lens according to the embodiments of the present invention has a remarkable inhibition effect against cell invasion, compared to the conventional single-piece type intraocular lens.

That is, it is found that the single-piece type intraocular lens according to the embodiments of the present invention has an excellent prevention capability against the generation of the aftercataract. The reason appears that the single-piece type intraocular lens according to the embodiments of the present invention has the stepped part 11a, the optic portion 1 and the edge part 12a are formed of soft acrylic materials, and therefore the synergistic effect of an insulation effect by the stepped part and an adhesion effect by the adhesiveness of the soft acrylic material is exerted.

Note that in the embodiments described above, the optic portion 1 is formed of a soft acrylic material, and the support portions 2a and 2b are formed of PMMA. However, materials forming the optic portion 1 include silicon and hydrogel, etc. , also. In addition, materials forming the support portions 2a and 2b include polyamide and a rigid acrylic materials (including PMMA) etc,. Note that the support portions and the optic portion may be formed of the same materials.

### Industrial applicability

As described above, the present invention enables a single-piece type intraocular lens having excellent shape memorizing properties of a material itself, excellent in stability of the intraocular lens after operation, also excellent in productivity, and further capable of remarkably decreasing the possibility of the generation of an aftercataract.

## Claims

1. A single-piece type intraocular lens obtained by shape processing a material formed by integrally molding an optic portion forming material and a support portion forming material, comprising a stepped part provided in a boundary between the optic portion and the support portion of the posterior of the optic portion so as to be arranged in such a way that the surface of a part shifting to a region of the support portion from the region of the optic portion suddenly shifts in a direction of the anterior of the optic portion.

2. The single-piece type intraocular lens according to claim 1, wherein the stepped part has an edge part which is formed in a part of the optic portion side of the boundary part shifting to the support portion from the optic portion, having a stepped face connecting from the edge part to the support portion serving as a wall face nearly in parallel to the optical axis of the optic portion.

3. The single-piece type intraocular lens according to claim 2, wherein the surface of the posterior of the optic portion near the edge part is formed in a surface substantially orthogonal to the optical axis.

4. The single-piece type intraocular lens according to claim 2, wherein the surface of the posterior of the optic portion near the edge part is formed so as to rise toward the edge part in a posterior direction.

5. The single-piece type intraocular lens according to claim 2, wherein a part of the stepped face closer to the support portions is formed into an acute angle so as to be inclined in a direction of the optical axis closely to the center of the optical axis.

6. The single-piece type intraocular lens according to claim 2, wherein a part of the stepped face closer to the support portions is formed into an obtuse angle so as to be slightly inclined in a direction of the optical axis in a direction opposite to the center of the optical axis.

7. The single-piece type intraocular lens according to claim 2, wherein R (curved surface) is formed in a part of the stepped face closer to the support portions.

8. The single-piece type intraocular Lens according to claim 2, wherein the stepped face is formed into a concavo-convex face.

9. The single-piece type intraocular lens according to any one of the claims 1 to 7, wherein the stepped part has a step difference with height of 0.05mm or more.

10. The single-piece type intraocular lens according to any one of the claims 1 to 9, wherein an edge part located in a region of the optic portion of the stepped part is formed of a soft material.

11. The single-piece type intraocular lens according to any one of the claims 1 to 10, wherein the optic portion is made of a soft material, and the support portion is made of a hard material.

12. The single-piece type intraocular lens according to claim 11, wherein the soft material is a soft acrylic material, and the hard material is PMMA.

13. The single-piece type intraocular lens according to any one of the claims 1 to 12, wherein the optical surface of the posterior of the optic portion is formed into a concave shape.

14. A manufacturing method of the single-piece type intraocular lens for manufacturing the single-piece type intraocular lens according to any one of the claims 1 to 13, comprising:
preparing a raw material formed by integrally molding the optic portion forming material and the support portion forming material;
cutting the raw material, thereby forming a curved surface shape of the optical surfaces of both sides of the anterior and the posterior of the optic portion, and a front surface shape of the support portion located on both sides of the anterior and the posterior of the optic portion;
next, grooving a part where the stepped part is estimated to be formed, thereby forming a surface serving as a stepped face; and
next, forming by cutting a contour shape excepting the anterior and posterior of the optic portion, and the contour shape excepting the surface shape located on both sides of the anterior and posterior of the optic portion of the support portion.
